# EUROPÄISCHE PATENTSCHRIFT

(11) **EP 2 432 344 B1**
(45) Veröffentlichungstag und Bekanntmachung des Hinweises auf die Patenterteilung: **23.04.2014**
(21) Anmeldenummer: 10721678.0
(22) Anmeldetag: 07.05.2010
(51) Int. Cl.: A43B 3/00, A43B 7/24, A43B 21/26, A43B 21/433, A61B 5/103

(54) **SCHUH, INSBESONDERE SPORTSCHUH**
SHOE, IN PARTICULAR SPORTS SHOE
CHAUSSURE, EN PARTICULIER CHAUSSURE DE SPORT

(30) Priorität: 19.05.2009 DE 202009007220 U
(43) Veröffentlichungstag der Anmeldung: 28.03.2012
(73) Patentinhaber: PUMA SE, 91074 Herzogenaurach (DE)
(72) Erfinder: BUDER, Jens, 09127 Chemnitz (DE); ODENWALD, Stephan, 09125 Chemnitz (DE)
(74) Vertreter: Gosdin, Michael
(86) Internationale Anmeldenummer: PCT/EP2010/002810
(87) Internationale Veröffentlichungsnummer: WO 2010/133300

(56) Entgegenhaltungen:
- WO-A2-2009/027941
- DE-U1-202007 018 163
- FR-A1- 2 898 017
- US-A1- 2007 006 489

## Beschreibung

Die Erfindung betrifft einen Schuh, insbesondere einen Sportschuh, der ein Schuhoberteil und eine Sohle aufweist, wobei in der Sohle mindestens ein Stellelement angeordnet ist, mit dem ein Bereich der Auflagefläche für den Fuß des Trägers des Schuhs hinsichtlich der Geometrie der Auflagefläche zumindest in einem definierten Längenbereich und/oder Breitenbereich der Sohle relativ zur Lage der Auflagefläche der Sohle auf dem Boden beeinflusst werden kann.

Ein Schuh dieser Art ist beispielsweise aus der DE 20 2007 018 163 U1 bekannt. Hier ist vorgesehen, einen Sportschuh mit einem System auszustatten, mit dem das Pronations- bzw. Supinationsverhalten des Schuhs aktiv beeinflusst werden kann. Ähnliche Lösungen zeigen die US 2007/0006489 A1**,** die FR 2 898 017 A1 und die WO 2009/027941 A2**.**

Die Pronation ist eine Drehung des Fußes um die Achse des unteren Sprunggelenks, bei der der äußere Fußrand gehoben und der innere Fußrand gesenkt wird. Die Pronation wird auch als Einwärtsdrehung oder Einwärtskantung bezeichnet. Die normale Pronation des Fußes ist ein natürlicher Dämpfungsmechanismus und eine natürliche Bewegung nach innen beim Fußaufsatz. Allerdings knickt der Fußrand bei der sog. Überpronation stark nach innen ein und belastet damit die Bänder, Sehnen und Gelenke. Diese Überpronation kann verschiedenste Ursachen haben, wie zum Beispiel eine Fußfehlstellung, Übergewicht oder starke Ermüdung. Ebenfalls tritt die Überpronation gelegentlich bei Laufanfängern auf, da der Stützapparat des Fußes noch nicht ausreichend trainiert ist. An den Schuhen ist dann oft eine starke Abnutzung im medialen Bereich erkennbar.

Der der Pronation entgegengesetzte Mechanismus (auch Supination genannt) tritt beim Laufen seltener auf. Bei der Supination geht die Belastung in die entgegengesetzte richtung. Bei Laufschuhen ist dies oft durch eine höhere Abnutzung im lateralen Bereich (also an der Außenseite) erkennbar.

Demgemäß wird es bei modernen Sportschuhen angestrebt, über die an sich bekannten in die Sohle eingearbeiteten Pronationsstützen hinaus eine aktive Beeinflussung der Pronation vorzunehmen, was mit einem System, wie es in der genannten Schrift erläutert ist, möglich ist.

Hierfür sind in der genannten DE 20 2007 018 163 U1 Stellsysteme vorgesehen, mit denen die Höhe eines Stellelements in Vertikalrichtung so verändert werden kann, dass sich die Auflagefläche der Sohle, auf der der Fuß des Trägers des Schuhs aufliegt, relativ zur Auflagefläche der Sohle auf dem Boden verändert. Da das Stellelement seitlich versetzt zur Schuhlängsachse in der Sohle angeordnet ist, kann damit eine Kippung der Fuß-Auflagefläche vorgenommen werden, die gezielt so geregelt werden kann, dass ein gewünschte Pronations- bzw. Supinationsverhalten erreicht wird.

Zur Einstellung der effektiven vertikalen Höhe des Stellelements wird dieses von einer Energiequelle (Batterie) mit Energie versorgt, die entsprechend im Schuh untergebracht ist. Das Stellelement hat nämlich einen Aktuator, mit dem die Verstellung vorgenommen werden kann. Die Energieversorgung ist auch erforderlich, um eine Steuer- bzw. Regeleinrichtung mit Energie zu versorgen, die das von einem Sensor detektierte Signal in ein entsprechendes Stellsignal für das Stellelement umsetzt.

Es ist in diesem Zusammenhang wünschenswert, die Energiequelle, d. h. die Batterie, möglichst klein auszuführen, um Gewicht zu sparen.

Der Erfindung liegt daher die A u f g a b e zugrunde, einen Schuh, insbesondere einen Sportschuh, der eingangs genannten Art so fortzubilden, dass es möglich wird, mit geringerem Energiebedarf die benötigte Stellbewegung eines gattungsgemäßen Stellelements vorzunehmen, wobei dennoch eine effektive Beeinflussung des Pronations- bzw. Supinationsverhaltens sichergestellt werden soll.

Die Lösung dieser Aufgabe durch die Erfindung ist dadurch gekennzeichnet, dass das Stellelement ein den Fuß des Trägers unterstützendes Trägerelement aufweist, das um eine Achse relativ zu einem Grundteil schwenkbar angeordnet ist, wobei zwischen dem Trägerelement und dem Grundteil beiderseits der Achse je mindestens eine in ihrem Volumen veränderbare Hydraulikkammer angeordnet ist, die vollständig mit einem weitgehend inkompressiblen Medium gefüllt sind, wobei die mindestens zwei Hydraulikkammern fluidisch über eine Leitung miteinander verbunden sind, in der ein schaltbares Ventil angeordnet ist.

Das Stellelement ist dabei bevorzugt so im Schuh angeordnet, dass die Achse mit der Längsachse des Schuhs zusammenfällt. Dies ist allerdings nicht zwingend. Die Achse kann beispielsweise auch parallel neben der Längsachse des Schuhs verlaufen.

Das Stellelement ist dabei bevorzugt im Hinterfußbereich der Sohle angeordnet. Es ist mit Vorteil im hinteren Drittel der Erstreckung der Sohle in Richtung der Längsachse des Schuhs angeordnet. Hinsichtlich der Ausdehnung des Stellelements in Breitenrichtung ist zu sagen, dass es sich bevorzugt über mindestens 80% der Breite der Sohle erstreckt; es kann aber auch ein geringerer Wert realisiert werden.

Sowohl das Trägerelement als auch das Grundteil sind bevorzugt als Platte ausgebildet. Dies ist allerdings nicht zwingend. Weiter kann vorgesehen werden, dass ein sich in Richtung der Achse erstreckender Steg zwischen dem Grundteil und dem Trägerelement angeordnet ist, der als Kippgelenk wirkt.

Die Hydraulikkammern haben vorzugsweise einen länglichen Kammerteil, der sich versetzt zur Achse in Richtung derselben erstreckt. Die Hydraulikkammern sind zumeist mit Hydrauliköl gefüllt; tauglich ist aber jede inkompressible Flüssigkeit.

Das Stellelement ist frei von einem von einer Energiequelle versorgten Betätigungselement. Die Verstellung des Stellelements erfolgt ausschließlich durch die Energie, die aufgrund der Körpermasse des Trägers des Schuhs vom Fuß des Trägers auf das Stellelement eingebracht wird.

Der Schuh hat bevorzugt weiterhin ein System, das das mindestens eine Stellelement umfasst, zur automatischen Beeinflussung der Pronation des Fußes des Trägers des Schuhs. Dieses System zur automatischen Beeinflussung der Pronation weist zumeist einen zur Erfassung eines für die Pronation relevanten Parameters geeigneten Sensor, eine Energiequelle und eine Steuer- bzw. Regeleinrichtung auf. Das Ventil kann von der Steuer- bzw. Regeleinrichtung betätigt werden.

Das Ventil ist bevorzugt ein Proportionalventil; es sind aber auch andere Ventilarten denkbar, z. B. ein Stellventil. Es kann ferner als Zwei-WegeVentil ausgeführt sein; allerdings sind auch andere Wegeventile möglich.

Mit der vorgeschlagenen Lösung wird es möglich, effektiv auf die Geometrie der Sohle des Schuhs so Einfluss zu nehmen, dass das Pronationsverhalten des Schuhs gezielt beeinflusst werden kann. Dabei wird insbesondere die Winkelstellung der Auflagefläche der Sohle für den Fuß relativ zur Auflagefläche des Schuhs auf dem Boden so verändert, dass sich ein gewünschtes Pronationsverhalten ergibt. Die Auflagefläche der Sohle für den Fuß des Trägers wird also - relativ zum Boden - so gekippt, dass die Pronation ein gewünschtes Maß nicht überschreitet. Vorteilhaft ist bei der beschriebenen Lösung, dass dies ohne jeglichen Energieeintrag in das Stellelement möglich wird, abgesehen von der Energie, die zur Betätigung des Ventils erforderlich ist, die jedoch untergeordnet ist.

Demgemäß kann mit einer sehr kleinen Energiequelle (Batterie), die nur für die Steuerung- bzw. Regelungseinrichtung und für die Ventilbetätigung benötigt wird, das Pronationsverhalten effektiv beeinflusst werden. Die Energie für die Verstellung des Stellelements wird vielmehr aus der Energie gewonnen, die der Träger des Schuhs auf das Stellelement beim normalen Laufen abgibt.

Es sei betont, dass durch den Einsatz eines weitgehend inkompressiblen Fluids in den Hydraulikkammern das Feder- und Dämpfungsverhalten nicht beeinflusst wird, was bestimmungsgemäß auch nicht vorgesehen ist. Lediglich die Verstellung des Stellelements wird nach der vorgeschlagenen Ausgestaltung vorgenommen; das Stellelement selber ist weitgehend starr und trägt nicht zur Federung und Dämpfung der Sohle des Schuhs bei.

In der Zeichnung ist ein Ausführungsbeispiel der Erfindung dargestellt. Es zeigen:
- Fig. 1: einen Sportschuh in perspektivischer Ansicht,
- Fig. 2: ein in der Sohle des Sportschuhs untergebrachtes Stellelement in perspektivischer Ansicht,
- Fig. 3: das Stellelement in der Draufsicht, wobei ein oben angeordnetes plattenförmiges Trägerelement entfernt ist,
- Fig. 4a: schematisch dargestellt das Stellelement in der Vorderansicht, in Richtung einer Kippachse A betrachtet, wobei keine Schwenkung eines Trägerelements relativ zu einem Grundteil vorliegt,
- Fig. 4b: das Stellelement in der Ansicht nach Fig. 4a, wobei eine Schwenkung des Trägerelements relativ zum Grundteil in eine erste Schwenkrichtung vorliegt, und
- Fig. 4c: das Stellelement in der Ansicht nach Fig. 4a, wobei eine Schwenkung des Trägerelements relativ zum Grundteil in eine zweite Schwenkrichtung vorliegt.

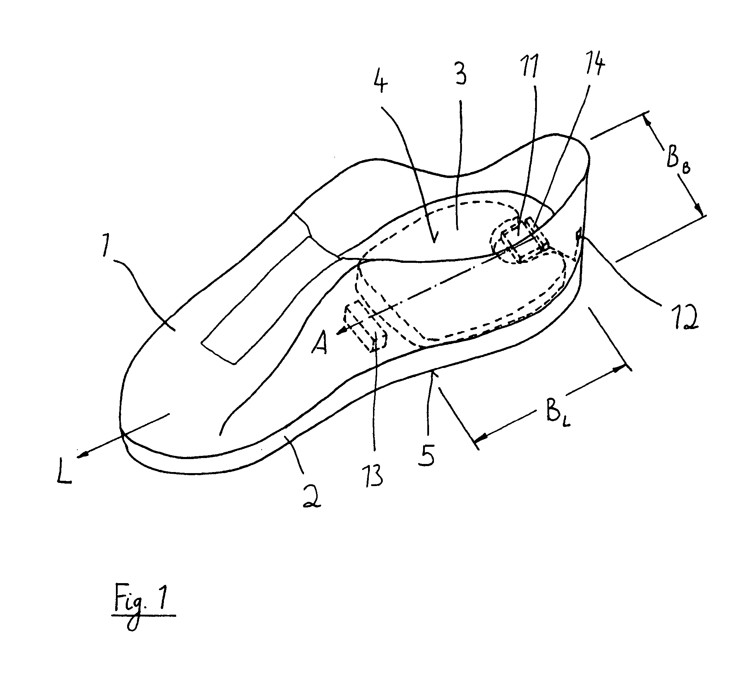

In Fig. 1 ist ein Sportschuh dargestellt, der in bekannter Weise im Wesentlichen aus einem Schuhoberteil 1 und einer Sohle 2 besteht. Der Schuh hat eine Längsachse L.

Der Schuh ist mit einem System ausgestattet, mit dem auf das Pronationsverhalten des Schuhs bzw. des Fußes des Trägers Einfluss genommen werden kann. Dieses System besteht im Wesentlichen aus einem Sensor 12, der einen Parameter erfassen kann, der für das Pronationsverhalten maßgeblich ist, beispielsweise die Drehgeschwindigkeit oder der Drehwinkel des Schuhs um eine definierte Achse. Weiterhin umfasst das System eine Steuer- bzw. Regeleinrichtung 14, die die vom Sensor 12 ermittelten Messwerte erhält. Das System umfasst weiterhin ein Stellelement 3, der von der Steuer- oder Regeleinrichtung 14 angesteuert wird. Das Stellelement 3 ist nachfolgend näher beschrieben. Wesentlich ist, dass es keinen eigenen Aktuator (z. B. Motor) aufweist, mit dem es eine Verstellbewegung ausführen könnte. Für die Energieversorgung der Steuer- bzw. Regeleinrichtung 14 ist indes eine Energiequelle 13 in Form einer Batterie vorgesehen. Da diese jedoch nur wenig Energie liefern muss, kann sie sehr klein ausgebildet sein. Die Steuer- bzw. Regeleinrichtung 14 kann auch an anderer Stelle platziert sein, als es Fig. 1 zeigt.

Das Stellelement 3 ist im Hinterfuß- oder Fersenbereich des Schuhs angeordnet. Es erstreckt sich im Ausführungsbeispiel etwa über das hintere Drittel der Sohle 2 gemessen in Richtung der Längsachse L. Die etwaigen Abmessungen des Stellelements 3 sind mit B_{L} für den Längenbereich und mit B_{B} für den Breitenbereich angegeben.

Bei der Betätigung des Stellelements 3 - nur während des Laufens und während der Bodenkontakphase - kippt eine Auflagefläche 4 für den Fuß des Trägers des Schuhs um eine Achse A relativ zur Auflagefläche 5 der Sohle 2 auf dem Boden. Die Auflagefläche 4 für den Fuß ist die Oberfläche der Sohle, auf der der Fuß des Trägers des Schuhs ruht.

Die Kipp- oder Schwenkachse A des Stellelements 3 ist vorliegend identisch mit der Längsachse L des Schuhs. Dies ist allerdings nicht zwingend.

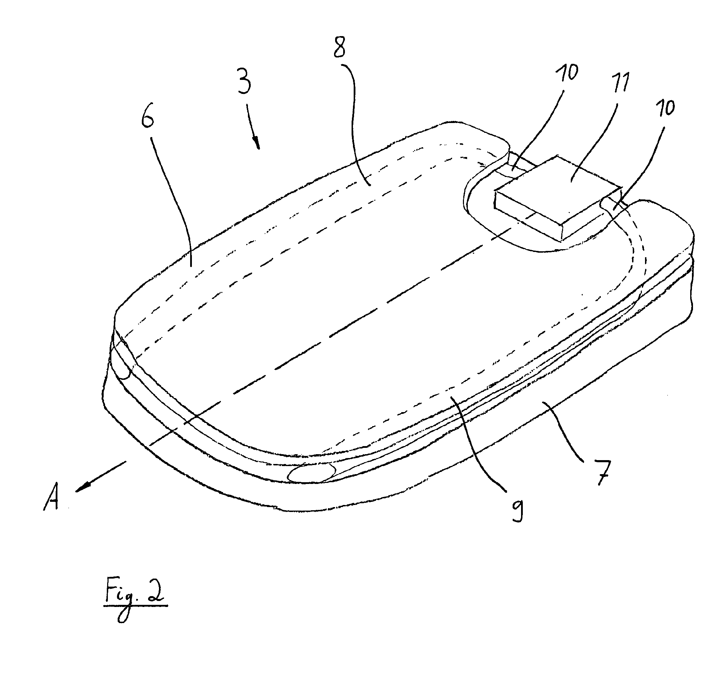

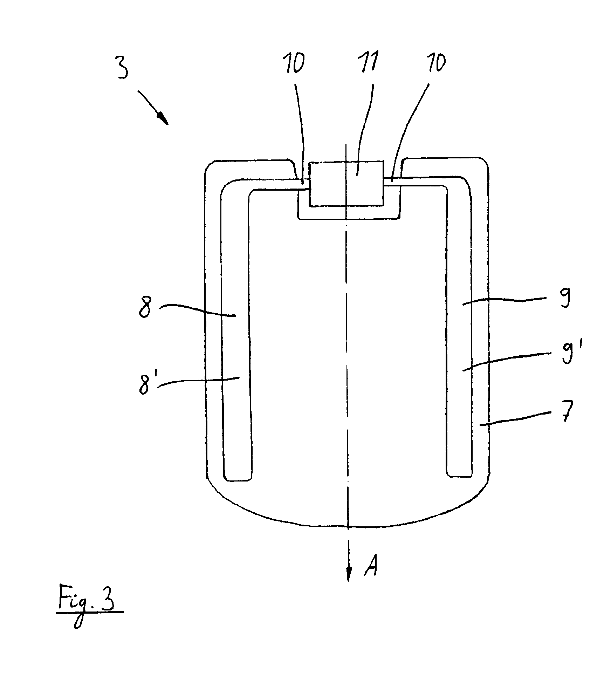

Details zum Aufbau des Stellelements 3 gehen Fig. 2 und aus Fig. 3 hervor. Das Stellelement 3 hat ein unten liegendes Grundteil 7, das fest in der Sohle 2 verankert sein kann, es kann beispielsweise in einer entsprechenden Ausnehmung in der Sohle 2 festgeklebt sein. Weiterhin hat das Stellelement 3 ein oben liegendes Trägerelement 6, auf dem der Fuß des Trägers - mittelbar getrennt durch eine nicht dargestellte Brand- und/oder Innensohle und ggf. durch eine Zwischensohle - aufliegt.

Zwischen dem Grundteil 7 und dem Trägerelement 6 sind zwei Hydraulikkammern 8 und 9 angeordnet, wie es sich aus der Zusammenschau der Figuren 2 und 3 ergibt. Die Hydraulikkammern 8, 9 sind z. B. mit Hydrauliköl oder einer anderen weitgehend inkompressiblen Flüssigkeit gefüllt. Da sowohl das Grundteil 7 als auch das Trägerelement 6 bevorzugt aus weitgehend festem Material bestehen (z. B. aus Kunststoff wie Polyamid oder auch aus Leichtmetall), stellt das gesamte Stellelement 3 eine weitgehend starre und nicht-elastische Struktur dar. Namentlich ist nicht angestrebt, dass das Stellelement eine federnde oder dämpfende Eigenschaft hat.

Entlang der Achse A kann sich - was nicht dargestellt ist - auch ein Steg erstrecken, der auf dem Grundteil 7 aufliegt und der eine Auflage für das Trägerelement 6 bildet.

Die Hydraulikkammern 8, 9 weisen zwei sich in Richtung der Achse A erstreckende Kammerteile 8' und 9' auf, die gleich weit beabstandet zur Achse A verlaufen (s. Fig. 3).

Die beiden Hydraulikkammern 8, 9 sind über eine Leitung 10 miteinander verbunden. In der Leitung 10 ist ein Ventil 11 angeordnet. Bei dem Ventil handelt es sich bevorzugt um ein Proportionalventil.

Das Ventil 11 wird, was in den Figuren nicht näher dargestellt ist, von der Steuer- bzw. Regeleinrichtung 14 mit Stellsignalen versorgt.

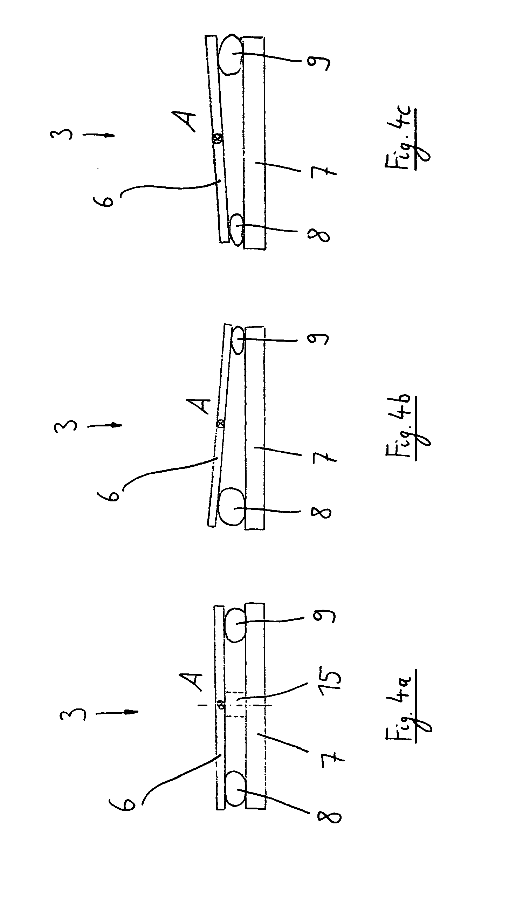

Die Steuer- bzw. Regeleinrichtung 14 entscheidet in Abhängigkeit der vom Sensor 12 detektierten Werte, ob und in welcher Weise auf das Stellelement 3 Einfluss genommen werden soll, um das Pronationsverhalten des Schuhs bzw. des Fußes des Trägers desselben zu beeinflussen. Die möglichen Wirkungsweisen, die sich aus entsprechenden Stellsignalen der Steuer- bzw. Regeleinrichtung 14 ergeben, sind in den Figuren 4a bis 4c - allerdings übertrieben dargestellt- illustriert.

In Fig. 4a ist zu sehen, dass keine Beeinflussung des Stellelements 3 vorgesehen ist. Demgemäß befindet sich das plattenförmig ausgebildete Trägerelement 6 parallel zum ebenfalls plattenförmig ausgebildeten Grundteil 7. Die beiden Hydraulikkammern 8, 9 sind demzufolge mit derselben Menge an Hydrauliköl gefüllt. Dies kann sich auch nicht ändern, da das Ventil 11 geschlossen gehalten wird. In diesem Zusammenhang sei allerdings angemerkt, dass es nicht zwingend ist, dass die beiden Kammern 8, 9 gleich groß sind. Es kommt primär auf die Höhe der Kammern an; das Volumen der Kammern 8, 9 kann unterschiedlich groß sein. Die Höhen und die Volumina der Kammern 8, 9 ergeben sich letztlich aus der Sohlengeometrie.

Mit gestrichelten Linien ist in Fig. 4a angedeutet, dass zwischen dem Grundteil 7 und dem Trägerelement 6 ein sich in Richtung der Achse A erstreckender Steg 15 vorgesehen sein kann, der eine Auflage für das Trägerelement 6 bildet.

Soll indes eine Beeinflussung des Fußes des Trägers erfolgen, um die Pronation zu verändern, wird das Ventil 11 von der Steuer- bzw. Regeleinrichtung 14 geöffnet, so dass es zu einem Fluss von Hydrauliköl von der einen Hydraulikkamer 8, 9 in die andere kommen kann.

In Fig. 4b ist zu sehen, dass ein Fluss von der Hydraulikkammer 9 in die Hydraulikkammer 8 erfolgte, so dass sich das Trägerelement 6 relativ zum Grundteil 7 schief gestellt hat, d. h. es ist um die Achse A geschwenkt worden.

Wesentlich ist, dass diese Schwenkung alleinig durch Öffnung des Ventils 11 im richtigen Moment erfolgte, als der Fuß des Trägers auf das Trägerelement 6 drückte. Diesen Moment zu ermitteln, ist der Steuer- bzw. Regeleinrichtung 14 möglich, da sie die entsprechenden Signale vom Sensor 12 erhält und daher "weiß", wann eine Beaufschlagung des Trägerelements 6 in der Art erfolgt, dass bei der Öffnung des Ventils 11 das gewünschte Umströmen von Hydraulikflüssigkeit von der Hydraulikkammer 9 in die Hydraulikkamer 8 erfolgt. Dieses Umströmen erfolgt in jedem Falle in der Bodenkontaktphase.

Den gegenteiligen Fall zeigt Fig. 4c. Hier wurde Hydraulikfluid von der Hydraulikkammer 8 in die Hydraulikkammer 9 analog zur oben beschriebenen Weise strömen gelassen.

Mit dem Stellelement 3 lässt sich also erreichen, dass die Winkelstellung der Auflagefläche 4 der Sohle für den Fuß des Trägers relativ zur Auflagefläche 5 auf dem Boden verändert wird. Auf das Pronationsverhalten kann so gezielt Einfluss genommen werden.

### Bezugszeichenliste:

- 1: Schuhoberteil
- 2: Sohle
- 3: Stellelement
- 4: Auflagefläche für den Fuß
- 5: Auflagefläche auf dem Boden
- 6: Trägerelement
- 7: Grundteil
- 8: Hydraulikkammer
- 8': Kammerteil
- 9: Hydraulikkammer
- 9': Kammerteil
- 10: Leitung
- 11: Ventil
- 12: Sensor
- 13: Energiequelle (Batterie)
- 14: Steuer- bzw. Regeleinrichtung
- 15: Steg

- L: Längsachse
- A: Achse
- B_{L}: Längenbereich
- B_{B}: Breitenbereich

## Patentansprüche

1. Schuh, insbesondere Sportschuh, der ein Schuhoberteil (1) und eine Sohle (2) aufweist, wobei in der Sohle (2) mindestens ein Stellelement (3) angeordnet ist, mit dem ein Bereich der Auflagefläche (4) für den Fuß des Trägers des Schuhs hinsichtlich der Geometrie der Auflagefläche (4) zumindest in einem definierten Längenbereich (B_{L}) und/oder Breitenbereich (B_{B}) der Sohle (2) relativ zur Lage der Auflagefläche (5) der Sohle (2) auf dem Boden beeinflusst werden kann,
**dadurch gekennzeichnet,**
**dass** das Stellelement (3) ein den Fuß des Trägers unterstützendes Trägerelement (6) aufweist, das um eine Achse (A) relativ zu einem Grundteil (7) schwenkbar angeordnet ist, wobei zwischen dem Trägerelement (6) und dem Grundteil (7) beiderseits der Achse (A) je mindestens eine in ihrem Volumen veränderbare Hydraulikkammer (8, 9) angeordnet ist, die vollständig mit einem weitgehend inkompressiblen Medium gefüllt sind, wobei die mindestens zwei Hydraulikkammern (8, 9) fluidisch über eine Leitung (10) miteinander verbunden sind, in der ein schaltbares Ventil (11) angeordnet ist.

2. Schuh nach Anspruch 1, **dadurch gekennzeichnet, dass** das Stellelement (3) so im Schuh angeordnet ist, dass die Achse (A) mit der Längsachse (L) des Schuhs zusammenfällt.

3. Schuh nach Anspruch 1 oder 2, **dadurch gekennzeichnet, dass** das Stellelement (3) im Hinterfußbereich der Sohle (2) angeordnet ist.

4. Schuh nach Anspruch 3, **dadurch gekennzeichnet, dass** das Stellelement (3) im hinteren Drittel der Erstreckung der Sohle (2) in Richtung der Längsachse (L) des Schuhs angeordnet ist.

5. Schuh nach einem der Ansprüche 1 bis 4, **dadurch gekennzeichnet, dass** sich das Stellelement (3) über mindestens 80% der Breite der Sohle (2) erstreckt.

6. Schuh nach einem der Ansprüche 1 bis 5, **dadurch gekennzeichnet, dass** das Trägerelement (6) als Platte ausgebildet ist.

7. Schuh nach einem der Ansprüche 1 bis 6, **dadurch gekennzeichnet, dass** das Grundteil (7) als Platte ausgebildet ist.

8. Schuh nach einem der Ansprüche 1 bis 7, **dadurch gekennzeichnet, dass** ein sich in Richtung der Achse (A) erstreckender Steg (15) zwischen dem Grundteil (7) und dem Trägerelement (6) angeordnet ist, der als Kippgelenk wirkt.

9. Schuh nach einem der Ansprüche 1 bis 8, **dadurch gekennzeichnet, dass** die Hydraulikkammer (8, 9) einen länglichen Kammerteil (8', 9') umfassen, der sich versetzt zur Achse (A) in Richtung der Achse (A) erstreckt.

10. Schuh nach einem der Ansprüche 1 bis 9, **dadurch gekennzeichnet, dass** die Hydraulikkammern (8, 9) mit Hydrauliköl gefüllt sind.

11. Schuh nach einem der Ansprüche 1 bis 10, **dadurch gekennzeichnet, dass** das Stellelement (3) frei von einem von einer Energiequelle versorgten Betätigungselement ist.

12. Schuh nach einem der Ansprüche 1 bis 11, **dadurch gekennzeichnet, dass** er weiterhin ein System, das das mindestens eine Stellelement (3) umfasst, zur automatischen Beeinflussung der Pronation des Fußes des Trägers des Schuhs aufweist.

13. Schuh nach Anspruch 12, **dadurch gekennzeichnet, dass** das System zur automatischen Beeinflussung der Pronation einen zur Erfassung eines für die Pronation relevanten Parameters geeigneten Sensor (12), eine Energiequelle (13) und eine Steuer- bzw. Regeleinrichtung (14) aufweist.

14. Schuh nach Anspruch 13, **dadurch gekennzeichnet, dass** das Ventil (11) von der Steuer- bzw. Regeleinrichtung (14) betätigt werden kann.

15. Schuh nach einem der Ansprüche 1 bis 14, **dadurch gekennzeichnet, dass** das Ventil (11) ein Proportionalventil, insbesondere ein Zwei-WegeVentil, ist.

## Claims

1. Shoe, especially sports shoe, comprising a shoe upper (1) and a sole (2), wherein at least one actuator (3) is arranged in the sole (2), by means of which a region of the contact surface (4) for the foot of the wearer of the shoe can be affected with regard to the geometry of the contact surface (4) at least in a defined length range (B_{L}) and/or width range (B_{B}) of the sole (2) relatively to the position of the contact surface (5) of the sole (2) on the ground,
**characterized in that**
the actuator (3) comprises a support element (6) supporting the foot of the wearer, which is pivotally disposed relatively to a base part (7) around an axis (A), wherein at least one hydraulic chamber (8, 9) having a variable volume is arranged on each side of the axis (A) between the support element (6) and the base part (7), which are completely filled with a substantially incompressible medium, wherein the at least two hydraulic chambers (8, 9) are fluidically connected to each other by means of a conduit (10) in which a switchable valve (11) is disposed.

2. Shoe according to claim 1, **characterized in that** the actuator (3) is arranged in the shoe in such a manner that the axis (A) coincides with the longitudinal axis (L) of the shoe.

3. Shoe according to claim 1 or 2, **characterized in that** the actuator (3) is arranged in the rear foot region of the sole (2).

4. Shoe according to claim 3, **characterized in that** the actuator (3) is arranged in the rearmost third of the extension of the sole (2) in the direction of the longitudinal axis (L) of the shoe.

5. Shoe according to one of claims 1 to 4, **characterized in that** the actuator (3) extends along at least 80% of the width of the sole (2).

6. Shoe according to one of claims 1 to 5, **characterized in that** the support element (6) is formed as a panel.

7. Shoe according to one of claims 1 to 6, **characterized in that** the base part (7) is formed as a panel.

8. Shoe according to one of claims 1 to 7, **characterized in that** a bar (15) is arranged between the base part (7) and the support element (6) extending in the direction of the axis (A) which acts as a tilting joint.

9. Shoe according to one of claims 1 to 8, **characterized in that** the hydraulic chambers (8, 9) comprise an elongated chamber part (8', 9') which extends offset to the axis (A) in the direction of the axis (A).

10. Shoe according to one of claims 1 to 9, **characterized in that** the hydraulic chambers (8, 9) are filled with hydraulic oil.

11. Shoe according to one of claims 1 to 10, **characterized in that** the actuator (3) is devoid of any actuating element provided by an energy source.

12. Shoe according to one of claims 1 to 11, **characterized in that** it further has a system which comprises the at least one actuator (3) for automatically influencing the pronation of the foot of the wearer of the shoe.

13. Shoe according to claim 12, **characterized in that** the system for automatically influencing the pronation comprises a suitable sensor (12) for detecting of a parameter which is relevant for the pronation, an energy source (13) and a control device and closed-loop control device (14) respectively.

14. Shoe according to claim 13, **characterized in that** the valve (11) can be actuated by the control device and closed-loop control device (14) respectively.

15. Shoe according to one of claims 1 to 14, **characterized in that** the valve (11) is a proportional valve, especially a 2-way valve.

## Revendications

1. Chaussure, en particulier chaussure de sport, qui présente une partie supérieure de chaussure (1) et une semelle (2), au moins un élément de réglage (3) étant disposé dans la semelle (2), avec lequel une région de la surface d'appui (4) pour le pied de l'utilisateur de la chaussure peut être influencée en termes de géométrie de la surface d'appui (4) au moins dans une région de longueur définie (B_{L}) et/ou dans une région de largeur définie (B_{B}) de la semelle (2) par rapport à la position de la surface d'appui (5) de la semelle (2) sur le sol,
**caractérisée en ce que**
l'élément de réglage (3) présente un élément de support (6) supportant le pied de l'utilisateur, lequel est disposé de manière à pouvoir pivoter autour d'un axe (A) par rapport à une partie de base (7), au moins une chambre hydraulique (8, 9) respective de volume variable étant disposée entre l'élément de support (6) et la partie de base (7) de chaque côté de l'axe (A), laquelle chambre hydraulique est complètement remplie d'un milieu essentiellement incompressible, les au moins deux chambres hydrauliques (8, 9) étant connectées l'une à l'autre fluidiquement par le biais d'une conduite (10) dans laquelle est disposée une valve commutable (11).

2. Chaussure selon la revendication 1, **caractérisée en ce que** l'élément de réglage (3) est disposé dans la chaussure de telle sorte que l'axe (A) coïncide avec l'axe longitudinal (L) de la chaussure.

3. Chaussure selon la revendication 1 ou 2, **caractérisée en ce que** l'élément de réglage (3) est disposé dans la région de la semelle (2) pour l'arrière du pied.

4. Chaussure selon la revendication 3, **caractérisée en ce que** l'élément de réglage (3) est disposé dans le tiers arrière de l'étendue de la semelle (2) dans la direction de l'axe longitudinal (L) de la chaussure.

5. Chaussure selon l'une quelconque des revendications 1 à 4, **caractérisée en ce que** l'élément de réglage (3) s'étend sur au moins 80 % de la largeur de la semelle (2).

6. Chaussure selon l'une quelconque des revendications 1 à 5, **caractérisée en ce que** l'élément de support (6) est réalisé sous forme de plaque.

7. Chaussure selon l'une quelconque des revendications 1 à 6, **caractérisée en ce que** la partie de base (7) est réalisée sous forme de plaque.

8. Chaussure selon l'une quelconque des revendications 1 à 7, **caractérisée en ce qu'**une nervure (15) s'étendant dans la direction de l'axe (A) est disposée entre la partie de base (7) et l'élément de support (6), laquelle sert d'articulation basculante.

9. Chaussure selon l'une quelconque des revendications 1 à 8, **caractérisée en ce que** les chambres hydrauliques (8, 9) comprennent une partie de chambre allongée (8', 9') qui s'étend de manière décalée par rapport à l'axe (A) dans la direction de l'axe (A).

10. Chaussure selon l'une quelconque des revendications 1 à 9, **caractérisée en ce que** les s chambres hydrauliques (8, 9) sont remplies d'huile hydraulique.

11. Chaussure selon l'une quelconque des revendications 1 à 10, **caractérisée en ce que** l'élément de réglage (3) est exempt d'élément d'actionnement alimenté par une source d'énergie.

12. Chaussure selon l'une quelconque des revendications 1 à 11, **caractérisée en ce qu'**elle présente en outre un système qui comprend au moins un élément de réglage (3) pour influencer de manière automatique la pronation du pied de l'utilisateur de la chaussure.

13. Chaussure selon la revendication 12, **caractérisée en ce que** le système pour influencer automatiquement la pronation présente un capteur (12) approprié pour détecter un paramètre relatif à la pronation, une source d'énergie (13) et un dispositif de commande ou de régulation (14).

14. Chaussure selon la revendication 13, **caractérisée en ce que** la valve (11) peut être actionnée par le dispositif de commande ou de régulation (14).

15. Chaussure selon l'une quelconque des revendications 1 à 14, **caractérisée en ce que** la valve (11) est une valve proportionnelle, en particulier une valve à deux voies.
